# FASCICULE DE BREVET EUROPEEN

(11) **EP 0 970 043 B1**
(45) Date de publication et mention de la délivrance du brevet: **07.11.2001**
(21) Numéro de dépôt: 98908162.5
(22) Date de dépôt: 10.02.1998
(51) Int. Cl.: C07C 229/48, A61K 31/195

(54) **COMPOSES CONSTITUANT NOTAMMENT DES EFFECTEURS DE RECEPTEURS DU SYSTEME NERVEUX CENTRAL SENSIBLES AUX AMINO ACIDES NEUROEXCITATEURS, LEUR PREPARATION ET LEURS APPLICATIONS BIOLOGIQUES**
VERBINDUNGEN VERWENDBAR ALS EFFEKTOREN VON NEUROEXCITATORISCHEN AMINOSÄURE-EMPFINDLICHEN REZEPTOREN DES ZENTRALNERVENSYSTEMS, IHRE HERSTELLUNG AND IHRE BIOLOGISCHEN VERWENDUNGEN
COMPOUNDS CONSISTING IN PARTICULAR OF EFFECTORS OF THE CENTRAL NERVOUS SYSTEM RECEPTORS SENSITIVE TO EXCITATORY AMINO ACIDS, PREPARATION AND BIOLOGICAL APPLICATIONS

(30) Priorité: 11.02.1997 FR 9701573
(43) Date de publication de la demande: 12.01.2000
(73) Titulaire: CENTRE NATIONAL DE LA RECHERCHE SCIENTIFIQUE, 75016 Paris Cédex (FR)
(72) Inventeur: ACHER, Francine, F-92420 Vaucresson (FR); AZERAD, Robert, F-91130 Ris Orangis (FR); PIN, Jean, Philippe, F-34000 Montpellier (FR)
(74) Mandataire: Peaucelle, Chantal
(86) Numéro de dépôt international: FR9800256
(87) Numéro de publication internationale: WO9834907

(56) Documents cités:
- WO-A-94/27602
- US-A- 5 500 420
- PETERSON, N. L. ET AL: "Characterization of retinal and hippocampal L-AP4 receptors using conformationally constrained AP4 analogs" BRAIN RES. (1991), 568(1-2), 15-23 CODEN: BRREAP;ISSN: 0006-8993, 1991, XP002042768

## Description

L'invention a pour objet des composés constituant notamment des effecteurs de récepteurs du système nerveux central sensibles aux amino acides neuroexcitateurs (EAA), en particulier au glutamate (Glu), leur préparation et leurs applications biologiques.

Elle concerne plus particulièrement des composés agonistes, antagonistes ou agonistes inverses de ces récepteurs.

On sait que le glutamate est impliqué dans de nombreuses fonctions cérébrales.

On attribue donc aux récepteurs glutamatergiques des rôles importants, notamment dans la conduction de l'influx nerveux, la plasticité synaptique, le développement du système nerveux central, l'apprentissage et la mémoire.

Le glutamate est également la principale neurotoxine endogène, étant responsable de la mort neuronale observée après ischémie, hypoxie, crise épileptique ou traumatismes du cerveau. Il n'est donc pas surprenant que les récepteurs du glutamate soient considérés comme impliqués dans divers troubles du système nerveux central et maladies neurodégénératives.

On mesure donc l'intérêt de disposer de moyens pour moduler les effets du glutamate dans le système nerveux central en utilisant des récepteurs glutamatergiques comme cibles.

Deux types principaux de récepteurs glutamatergiques ont été caractérisés : les récepteurs ionotropiques et les récepteurs métabotropiques. Les récepteurs ionotropiques sont des canaux cationiques actionnés par le glutamate et directement responsables d'une dépolarisation rapide des cellules post-synaptiques. Ils sont composés de différentes sous-unités et classés en trois groupes selon leurs propriétés pharmacologiques et fonctionnelles. On distingue ainsi les récepteurs NMDA (acide N-méthyl-D-aspartique), AMPA (acide α-amino-3-hydroxy-5-méthyl-isoxazole-4-propionique) et kainate. Les récepteurs métabotropiques (mGluRs) ont été plus récemment mis en évidence (1985), et ont été moins bien étudiés en raison de l'absence d'effecteurs spécifiques, jusqu'à la découverte de l'acide 1-amino cyclopentane 1,3 dicarboxylique ou ACPD. Ils régulent l'activité des canaux ioniques ou d'enzymes produisant des seconds messagers via des protéines G liant le GTP. Bien qu'ils ne soient pas généralement impliqués directement dans la transmission synaptique rapide, ces récepteurs modulent l'efficacité de ces synapses en régulant soit les canaux ioniques post-synaptiques et leurs récepteurs, soit la libération ou la recapture du glutamate. Les mGluRs jouent donc un rôle important notamment dans l'induction de la potentiation à long terme (LTP) et la dépression à long terme (LDP) de la transmission synaptique, dans la régulation des réflexes baroceptifs, l'apprentissage dans l'espace, l'apprentissage moteur, l'intégration posturale et cinétique. Ils sont également probablement impliqués dans les maladies dégénératives aigües ou chroniques telles que l'épilepsie, la maladie d'Alzheimer, la maladie de Parkinson ou la chorée de Huttington.

Huit mGluRs ont été clonés à ce jour et peuvent être classés en 3 groupes selon leurs homologies de séquence, leurs propriétés pharmacologiques et leurs mécanismes de transduction du signal.

Les récepteurs métabotropiques présentent un grand intérêt comme cibles pour la modulation des effets du glutamate, mais leur rôle dans diverses réponses physiologiques impliquant le glutamate reste mal caractérisé à ce jour en raison de l'absence d'outils parfaitement spécifiques. Différents ligands, analogues de l'acide glutamique, ont été récemment décrits et sont utilisés pour la caractérisation pharmacologique et physiologique des récepteurs métabotropiques, mais s'avèrent insuffisamment sélectifs et ne permettent pas la différentiation parfaite des groupes ou, à l'intérieur d'un groupe, du sous-groupe auquel appartient le récepteur.

Il existe un besoin de nouvelles molécules permettant la différenciation des récepteurs ionotropiques et métabotropiques. Il est également important de disposer de molécules plus sélectives permettant à l'intérieur de la famille des récepteurs métabotropiques, la recherche et l'étude de ces récepteurs, la définition de structures bioactives propres à chacun d'eux, et l'élaboration de nouvelles molécules pharmacologiquement actives.

Dans ce contexte, les inventeurs ont étudié des analogues cycliques de l'acide glutamique à conformation restreinte et ont constaté que la présence de certains groupements dans des positions déterminées, avec les possibilités stéréochimiques résultantes, conduisait à des composés dotés d'activités renforcées et/ou nouvelles vis-à-vis des récepteurs glutamatergiques.

L'invention a donc pour but de fournir de nouveaux composés dotés d'effets avantageux vis-àvis de récepteurs du système nerveux central.

Elle vise également à fournir un procédé de synthèse de ces composés de mise en oeuvre aisée, exploitable à l'échelle industrielle.

Elle vise aussi la mise à profit des propriétés de ces nouveaux composés pour l'élaboration d'agents agonistes ou antagonistes de ces récepteurs, utilisables comme outils de recherche et d'étude, ou comme médicaments.

Les composés de l'invention sont caractérisés en ce qu'ils répondent à la formule (I) 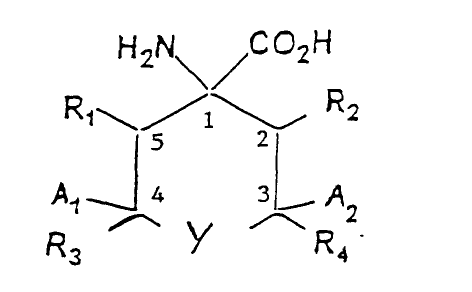 dans laquelle
- R₁ à R₄, identiques ou différents les uns des autres, représentent un atome d'hydrogène, un radical alkyle ou un radical aryle, ces radicaux étant le cas échéant eux-mêmes substitués, R₁ et R₂ ensemble pouvant en outre représenter un pont -(CH₂)ₘ-, où m est un entier de 1 à 5,
- A₁ et A₂ représentent tous deux l'un des radicaux -COOH, -SO₃H ou -PO₃H₂, ou leurs dérivés tels qu'esters ou amides,
- Y représente une chaîne hydrocarbonée -(CH₂)ₙ-, n étant nul ou un entier de 1 à 5, ou un hétéroatome O, N ou S, ou une combinaison linéaire de n groupes hydrocarbonés et au moins un hétéroatome O, N ou S.

Une famille préférée est formée de composés monocycliques. Il s'agit de composés de formule I dans laquelle Y est remplacé par une liaison simple entre les carbones en positions 3 et 4 et les carbones portant R₁ et R₂ ne sont pas reliés entre eux en formant l'un des ponts précités.

Dans une autre famille de l'invention, les composés sont également monocycliques et répondent à la formule I ci-dessus, dans laquelle les carbones portant R₁ et R₂ ne sont pas reliés entre eux en formant l'un des ponts précités, et Y représente une chaîne hydrocarbonée ou un hétéroatome comme défini ci-dessus.

Dans encore une autre famille de l'invention, les composés sont bicycliques et répondent à la formule I ci-dessus dans laquelle sont présents un pont hydrocarboné formé à partir de R1 et R2, ainsi que Y, ledit pont et Y étant tous deux définis comme indiqué ci-dessus.

Dans un groupe préféré de composés de ces différentes familles, A₁ et A₂ sont identiques. Dans des composés préférés de ce groupe A₁ et A₂ représentent tous deux un groupe carboxyle, ou les esters ou amides correspondants.

Dans un autre groupe, A₁ et A₂ sont tels que définis plus haut, mais diffèrent l'un de l'autre.

Des composés avantageux de ces familles et groupes comprennent des substituants R₁, R₂, et/ou R₃ et deux un groupe carboxyle, ou les esters ou amides correspondants.

Dans un autre groupe, A₁ et A₂ sont tels que définis plus haut, mais diffèrent l'un de l'autre.

Des composés avantageux de ces familles et groupes comprennent des substituants R₁, R₂, et/ou R₃ et R₄ représentant un radical alkyle ou aryle, ces radicaux étant le cas échéant substitués.

Par radical "alkyle", on entend conformément à l'invention un radical de 1 à 10 atomes de carbone, notamment de 1 à 5 atomes de carbone. Par radical "aryle", on entend un radical aromatique mono- ou polycyclique. Un radical aryle préféré est constitué par le radical phényle.

Les radicaux alkyle ou aryle peuvent être substitués, par exemple, par un atome d'halogène ou un groupe hydroxyle.

Dans d'autres composés avantageux, R₁ et R₂ et/ou R₃ et R₄ représentent un atome d'hydrogène.

Les composés définis ci-dessus peuvent exister comme molécules achirales ou chirales, sous forme de différents diastéréoisomères, éventuellement comme racémique, le cas échéant sous forme de l'un des énantiomères.

L'invention vise en particulier les acides 1-amino- cyclopentane -1, 3, 4 tricarboxyliques ACPT-I, ACPT-II, ACPT-IIIa et ACPT-IIIb qui répondent respectivement aux formules (II), (III), (IVa) et (IVb) suivantes :

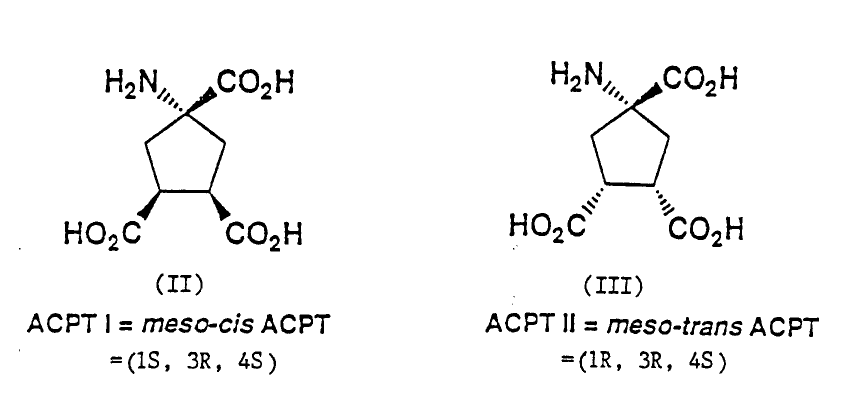

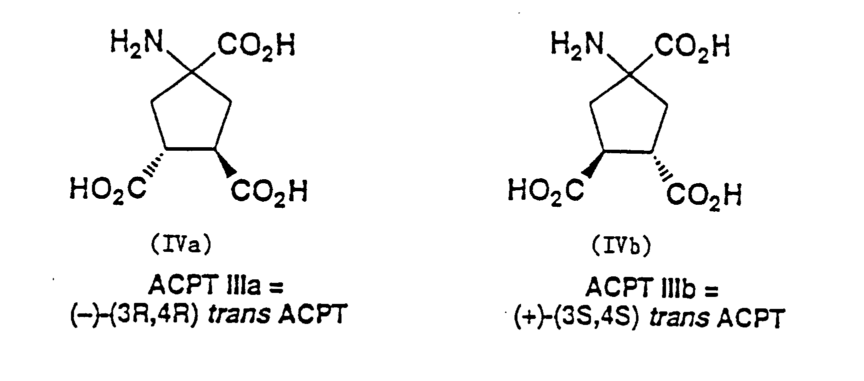

L'invention vise également un procédé de synthèse des composés définis ci-dessus.

Cette synthèse, avantageusement effectuée selon la réaction de Bucherer-Bergs ou celle de Strecker, est caractérisée en ce qu'elle comprend
- en opérant selon la technique de Bucherer-Bergs, la réaction d'une cétone de formule (V) 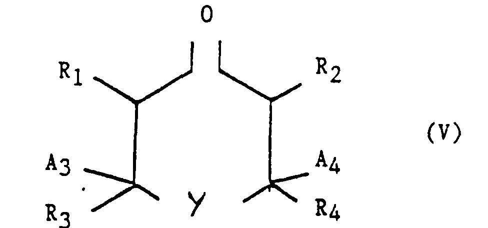 dans laquelle R₁ à R₄ et n sont tels que définis dans la formule I et A₃ et A₄ représentent un groupe -COOR₅, PO₃(R₅)₂, PO₃HR₅, ou SO₃R₅, où R₅ est un atome d'hydrogène ou un radical alkyle,
   avec un cyanure et un carbonate basique d'ammonium, ou
- en opérant selon la technique de Strecker, la réaction de la cétone (V) avec un sel d'ammonium et un cyanure, puis l'addition de carbonate d'ammonium au mélange réactionnel, ces réactions étant réalisées dans des conditions conduisant à une hydantoine de formule (VI) 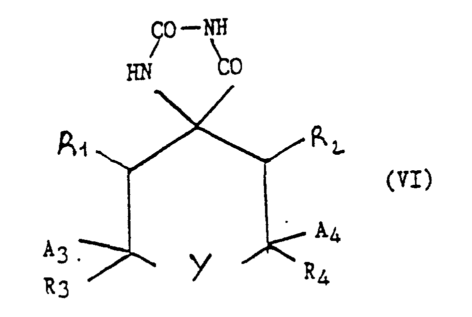
- suivie de l'hydrolyse de l'hydantoine ainsi obtenue, ou d'une hydantoine dérivée dans laquelle les groupes -NH sont bloqués par des groupes protecteurs, cette hydrolyse conduisant à
- l'obtention d'un mélange renfermant divers diastéréoisomères,
- à la séparation des différents composés, et au dédoublement des racémiques si souhaité.

Dans un mode de réalisation, on fait réagir la cétone (V) avec un carbonate d'ammonium et un cyanure, et après une étape de chauffage, le milieu réactionnel résultant est acidifié.

La cétone est en solution dans un solvant organique de type alcool, en particulier dans du méthanol.

Le carbonate d'ammonium est utilisé dans un excès de 3 à 10 équivalents par rapport à la cétone, de préférence de 5 équivalents environ.

Comme cyanure approprié, on citera les cyanures minéraux, comme le cyanure de sodium ou celui de potassium. Le cyanure est mis en oeuvre à raison de 1 équivalent ou en léger excès par rapport à la cétone.

Le mélange réactionnel obtenu par addition, des composés ci-dessus, de préférence sous forme d'une solution aqueuse, à la cétone est porté à une température supérieure à 40°C et inférieur à 90° C environ.

On peut procéder au chauffage du mélange dans une première gamme de température, avec réfrigérant, puis poursuivre à une température supérieure, sans réfrigérant, de manière à éliminer le sel d'ammonium en excès.

Des résultats satisfaisants ont été ainsi obtenus en chauffant le mélange pendant environ 8 à 15 h à environ 50 à 70°C, en particulier à 55-60°C environ, avec réfrigérant, puis à une température de l'ordre de 75 à 90°C, pendant 0,5 à 3 heures, notamment d'environ 80°C, pendant 1 heure, sans réfrigérant.

Le mélange réactionnel est ensuite acidifié. On soumet par exemple le mélange à agitation après addition de l'acide, ce qui conduit à choisir un temps de réaction de l'ordre de 10 à 60 min, notamment d'environ 15 à 30 min. L'acide est avantageusement ajouté de manière à obtenir un pH de 3 à 4 environ.

Dans un autre mode de réalisation, l'obtention des hydantoines est réalisée en ajoutant tout d'abord à la cétone (V) de départ un sel d'ammonium, par exemple du chlorure d'ammonium, et un cyanure pour former le dérivé aminonitrile correspondant, puis en ajoutant du carbonate d'ammonium, suivi, après réaction, de l'acidification du milieu.

La cétone est alors plus spécialement mise en oeuvre en solution aqueuse. Le sel d'ammonium et le cyanure, également en solution aqueuse, sont ajoutés à la cétone. En opérant à température ambiante, et sous agitation, la réaction conduisant à l'obtention des amino nitriles correspondants s'effectue en 1 à 3 jours environ.

Le carbonate d'ammonium est alors ajouté au mélange réactionnel puis, après plusieurs heures d'agitation, la solution obtenue est acidifiée à un pH d'environ 3 à 4.

Le passage des hydantoïnes aux amino acides correspondants est réalisé par hydrolyse.

Dans une variante, on ajoute une solution acide ou basique à l'hydantoine précédemment obtenue.

De préférence la solution acide renfermant l'hydantoine est préalablement évaporée à sec et le résidu soumis à une étape d'extraction pour éliminer au moins une partie des sels.

L'extraction est réalisée par exemple à l'aide d'un alcool tel que le méthanol.

Le filtrat récupéré est évaporé et dissous dans la solution acide ou basique et soumis à une étape de chauffage. On utilise avantageusement de l'acide chlorhydrique ou de l'acide sulfurique 2 à 8 N, de préférence 6 N, ou de la soude ou de la potasse 2 à 8 N, de préférence 6 N, ou de la baryte en solution saturée.

En opérant dans un récipient clos à une température supérieure à 50°C, en particulier de 100 à 130°C environ, notamment de l'ordre de 110° ou 120°C, le traitement est effectué sur plusieurs jours, notamment de 2 à 6 jours.

Dans une autre variante, les groupes-NH des hydantoïnes sont tout d'abord bloqués par des groupements protecteurs, puis le dérivé ainsi obtenu est soumis à une étape d'hydrolyse basique ménagée.

La solution d'hydantoïne obtenue à l'issue de l'étape de synthèse précédemment exposée est évaporée à sec et l'hydantoine résultante estérifiée, par exemple par HCl 1N sec dans du méthanol. Après évaporation, le résidu est repris dans un solvant organique tel que l'acétate d'éthyle, et lavée par une solution de sel saturée. La phase organique renferme les esters de l'hydantoine.

L'hydantoine estérifiée est soumise à une étape d'évaporation puis de séchage, et dissoute dans un solvant organique tel que CH₃CN.

Pour obtenir le blocage des groupes -NH, on procède à la formation d'un alkyl-carbamate en présence de diméthyl-aminopyridine (DMAP).

Cette protection est effectuée de manière particulièrement avantageuse sous forme de dérivés butyloxycarbonyl en utilisant le di-t-butyldicarbonate (Boc).

Après environ 1 à 2 heures de réaction, le composé bloqué est récupéré, par exemple après passage sur une colonne de chromatographie.

L'hydantoïne ainsi protégée est dissoute dans de l'acétonitrile, et son hydrolyse est réalisée en ajoutant une base telle que de l'hydroxyde de lithium, de préférence sous forme d'une solution de concentration 1N, puis on neutralise avec un acide tel que HCl 1N, on évapore à sec et on traite le résidu avec une solution acide constituée par exemple par HCl/CH₃COOH.

La séparation des diastéréoisomères du mélange d'amino acides est avantageusement effectuée par chromatographie sur colonne échangeuse d'anions. Avantageusement, cette étape est précédée d'une purification réalisée par chromatographie d'échanges de cations.

Différents choix seront opérés pour la mise en oeuvre du procédé de l'invention :
- celui de la réaction de Bucherer-Bergs ou celle de Strecker est dicté par le choix d'une configuration préférentielle cis ou trans (position du groupe -COOH en position 1 par rapport à A₁ et A₂), (cas par exemple des amino acides méso),
- celui de la stéréochimie de la cétone de départ par la configuration finale souhaitée pour les composés à synthétiser (cas des amino acides où A₁ et A₂ ont une configuration relative trans par exemple).

L'étude des activités biologiques des composés de l'invention vis-à-vis des récepteurs du système nerveux central sensibles au glutamate a mis en évidence leur spécificité vis-à-vis des récepteurs métabotropiques.

Les tests réalisés à l'aide de ces composés ont en effet montré qu'ils ne possèdent pas d'activité agoniste ou antagoniste sur les récepteurs ionotropiques (essais effectués à 1 mM sur des cellules granulaires du cervelet, normalement activées par le NMDA ou le kainate 100 *µ*M).

En revanche, ces composés exercent un effet agoniste ou antagoniste vis-à-vis des récepteurs métabotropiques, comme le montrent les résultats donnés dans les exemples. Selon un aspect revêtant un intérêt majeur, cette réaction est sélective, pour un composé donné, par rapport à un sous-groupe de récepteurs.

Ainsi, l'ACPT-I est un agoniste de mGluR4. L'ACPT-II est un antagoniste général de tous les mGluR testés (mGluR1a, mGluR2 et mGluR4a) et est doté de propriétés agonistes inverses sur mGluR1. L'ACTP-IIIa est un antagoniste de mGluR4, et l'ACTP-IIIb, un agoniste de mGluR4, ces deux ACTP-IIIa et IIIb étant des antagonistes de mGluR1 et mGluR2.

L'invention vise donc l'application des composés définis ci-dessus comme agents agonistes ou antagonistes spécifiques de récepteurs glutamatergiques métabotropiques.

Ces agents sont plus spécialement utilisables comme outils pour étudier spécifiquement chacun des groupes et sous-groupes de récepteurs métabotropiques en les différentiant et en permettant ainsi de définir leurs rôles physiologiques spécifiques respectifs.

Des applications basées sur l'utilisation de tels outils comprennent la mise en contact des composés définis ci-dessus avec des cultures cellulaires exprimant lesdits récepteurs, dans des conditions permettant la réalisation de la réaction agoniste, antagoniste ou agoniste inverse à étudier.

Les effets avantageux des composés de l'invention s'accompagnent d'une grande innocuité comme l'ont montré les essais réalisés sur des souris.

L'invention vise donc la mise à profit des propriétés de ces composés sous forme acide, ou en tant que pro-drogues, de leurs amides ou esters, pour l'élaboration de compositions pharmaceutiques.

Les compositions pharmaceutiques de l'invention sont caractérisées en ce qu'elles renferment une quantité efficace d'au moins un composé tel que défini ci-dessus, en association avec un véhicule pharmaceutique inerte.

Ces compositions renferment le cas échéant des principes actifs d'autres médicaments.

Les compositions de l'invention sont particulièrement appropriées pour moduler l'effet du glutamate, par exemple dans le traitement de troubles de la mémoire, de la démence sénile, de l'épilepsie, de la maladie d'Alzheimer, de la maladie de Parkinson, de neuropathies périphériques et de troubles moteurs.

Les conditionnements en vue de la vente, en particulier l'étiquetage et les notices d'emploi, et avantageusement l'emballage sont élaborés en fonction de l'application thérapeutique prévue.

Les compositions pharmaceutiques de l'invention sont administrables sous différentes formes, plus spécialement par voie orale ou injectable, ou encore nasale.

Pour l'administration par voie orale, on a recours en particulier à des comprimés, pilules, tablettes, gélules, gouttes ou encore à des liposomes. Ces compositions renferment avantageusement de 1 à 100 mg de principe actif par unité de prise, de préférence de 2,5 à 50 mg.

D'autres formes d'administration comprennent des solutions injectables par voie intra-veineuse, sous-cutanée ou intra-musculaire, élaborées à partir de solutions stériles ou stérilisables. Il peut s'agir également de suspensions ou d'émulsions.

Ces formes injectables renferment par unité de prise de 0,5 à 50 mg de principe actif, de préférence de 1 à 30 mg.

A titre indicatif, la posologie utilisable chez l'homme correspond aux doses suivantes : ainsi on administre par exemple au patient 5 à 300 mg/jour, en une ou plusieurs prises.

D'autres caractéristiques et avantages de l'invention sont donnés dans les exemples qui suivent dans lesquels il est fait référence aux figures 1 à 5 qui représentent respectivement
- les figures 1a à 1c, l'activation de récepteurs mGluRs par des ACPT,
- la figure 2, l'inhibition par les ACPT de la formation de IP induite par Glu.
- les figures 3a à 3c, la détermination de l'effet antagoniste et de la constante d'inhibition de l'ACPT-II, à différentes concentrations, sur les mGluRs, et les figures 3d à 3f, les représentations graphiques de Shild correspondantes,
- les figures 4a à 4c, l'effet antagoniste des isomères de l'ACPT-III sur les mGluRs, et
- la figure 5, l'effet agoniste inverse des ACPT sur mGluR1a.

Ces exemples se rapportent aux ACPT-I (1S, 3R, 4S), ACPT-II (1R, 3R, 4S), (±)-ACPTIII, (3R, 4R)-ACPT-III, et (3S,4S)-ACPT-III. Ces composés ont été synthétisés par la réaction de Bucherer-Bergs ou celle de Strecker, à partir des cétoacides 5, (±)-8, (3R,4R)-8 ou (3S,4S)-8(schémas 1a et 1b, 2 et 3 ci-après), suivie de l'hydrolyse des hydantoïnes formées (schéma 4), et de la séparation des ACPT par chromatographie sur échangeur d'anions.

### - Synthèse du cétodiester 5

On opère selon Gais, H.-J. ; Bülow, G. ; Zatorski, A.; Jentsch, M. ; Maidonis, P. ; Hemmerle, H.J., *J*. *Org*. *Chem*. 1989, 54, 5115-5122 ; Hemmerle, H. Dissertation, Universität Freiburg im Brisgau, Germany, 1990.

Cette synthèse est illustrée sur le schéma la suivant : 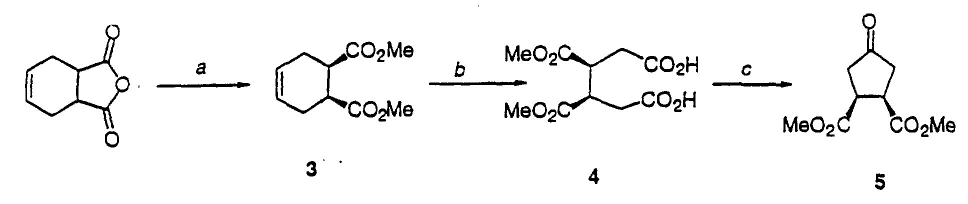 a: H₂SO₄/MeOH; b: KMnO₄; c:AcONa/Ac₂O, 140°C.

### - Synthèse du cétodiester (±) 8

On opère selon Rosenquist, A.; Kvarnstrôm, I.; Svensson, S.C. T.; Classon, B.; Samuelsson, B. *Acta Chem. Scand.* 1992, 46, 1127-1129.

Cette synthèse est illustrée sur le schéma 1b suivant : 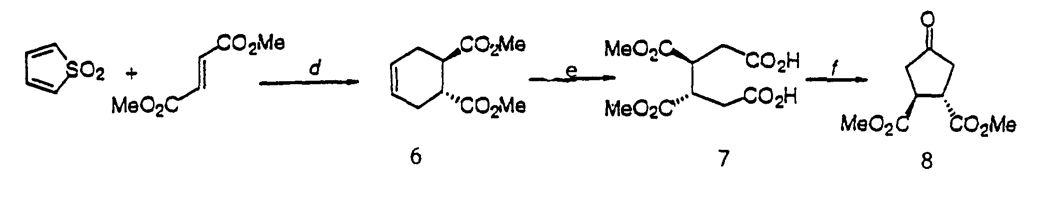 d:tube scellé, 100°C (Sample, T.E.; Hatch, L.F. *Org. Synth. Coll.* Vol. VI 1988, 454) ; e: KMnO₄ ; f: AcONa/Ac₂O, 140°C.

### - Synthèse des cétodiesters (3R, 4R)-8 et (3S,4S) -8.

On opère selon Suemune, H.; Tanaka, M.; Obaishi, H.; Sakai, K. *Chem. Pharm. Bull.* 1988, 36, 15-21; Rosenquist, A.; Kvarnström, I.; Svensson S. C.T. ; Classon, B.; Samuelsson, B. *Acta Chem*. *Scand.* 1992, 46, 1127-1129.

Cette synthèse est illustrée sur le schéma 2 suivant : 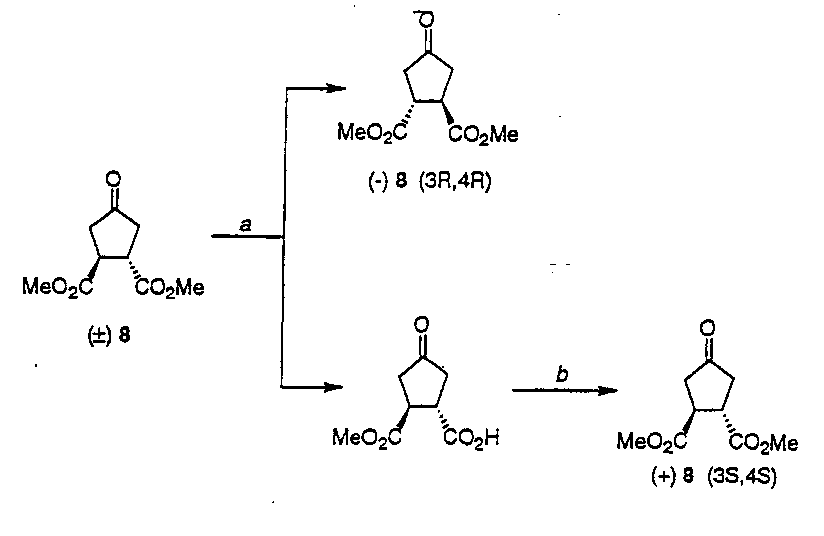 a: estérase de foie de porc, pH 7.0; b:CH₃N₂/CH₂Cl₂

### - Synthèse des hydantoines par la méthode de Bucherer-Bergs ou par la méthode de Strecker, via les aminonitriles correspondants.

La synthèse de Strecker permet d'obtenir, à partir du cis-cétoester 5, l'introduction du groupe aminé dans une proportion légèrement plus favorable au produit comportant le groupement aminé en cis par rapport aux carboxylates que la réaction de Bucherer-Bergs (ACPT-I, environ 30 % au lieu de 20 % , voir exemple 4)

Cette synthèse est illustrée par le schéma 3 suivant :

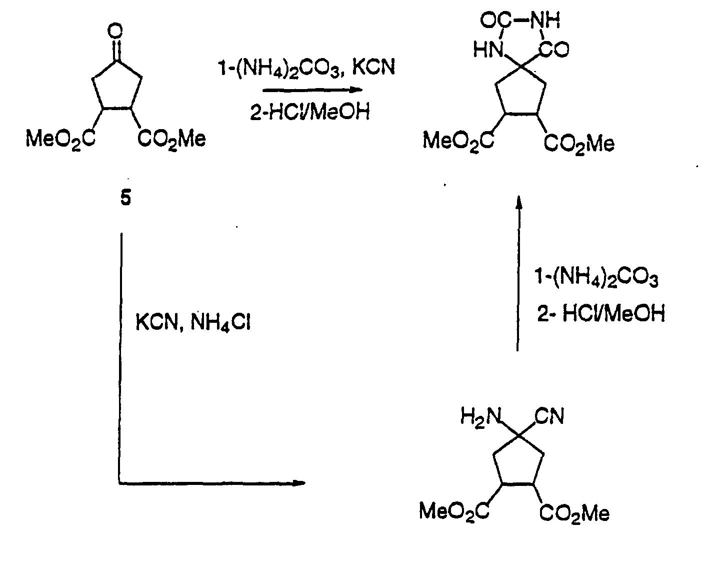

### - Passage des hydantoïnes aux amino acides correspondants.

L'hydrolyse directe des hydantoïnes conduit à une épimérisation significative d'un ou (des) groupes carboxyliques dans le sens cis----> trans (environ 25:75 à l'équilibre), pouvant même conduire à la racémisation d'un énantiomère trans. Pour obtenir une stéréochimie correcte et conforme à celle du cétoacide initialement employé, la méthode d'hydrolyse alcaline des dérivés diBoc de l'hydantoine (Kubik, S. ; Meissner, R.S. ; Rebek Jr., J. *Tetrahedron Lett.* 1994, 35, 6635-6638) doit être préférée, car elle ne produit qu'une épimérisation (ou racémisation) très minime (voir application au cas de l'hydantoine issue du cis-cétoester 5, exemple 4, ou des hydantoïnes issues des cétoesters trans énantiomériquement purs (3R, 4R)-8 ou (3S-4S)-8, exemples 6 et 7).

Cette synthèse est illustrée par le schéma 4 suivant :

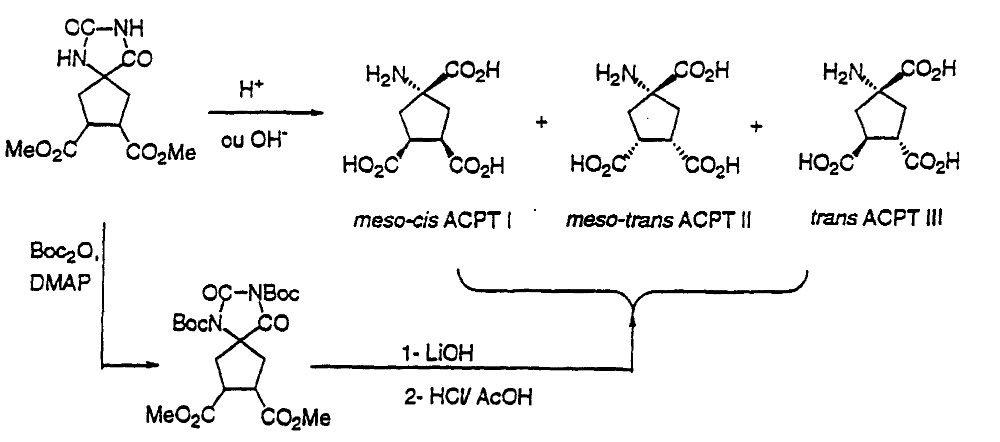

On rapporte ci-après les synthèses détaillées des ACPT à partir des cétodiesters.

### Exemple 1:

Au *cis*-cétodiester **5** (0,2 g, 1 mmol) dissous dans MeOH (1,5 ml) sont ajoutés (NH₄)₂CO₃ (0,48 g, 5 eq.) et KCN (0,077 g, 1,2 eq.) dissous dans l'eau (1,5 ml). Le mélange est chauffé une nuit à 55-60 °C avec réfrigérant, puis 1 h à 80 °C sans réfrigérant (afin d'éliminer (NH₄)₂CO₃ en excès) et acidifié avec une solution d'HCl concentré. La solution acide est agitée 0.5 h à température ambiante. Le résidu obtenu après évaporation à sec est extrait avec MeOH (pour éliminer une partie des sels), le filtrat évaporé (0,23 g) est dissous dans une solution d'HCl 6N (20 ml) dans une bouteille étanche à vis et chauffé à 110 °C pendant 5 jours. La solution est évaporée, diluée avec 200 ml d'eau et déposée à pH acide (environ pH 2-3) sur une colonne d'échangeur de cations Dowex 50X4 (H⁺, 20-50 mesh, 2 x 14 cm). La colonne est rinçée à l'eau (200ml) et le mélange des ACPT élué avec une solution d'ammoniaque 0,5 M. Les fractions positives à la ninhydrine sont évaporées (0,2 g, 0,92 mmol) et le mélange redissous dans de l'eau bouillie (200 ml) est déposé à pH basique (environ pH 9-10) sur une colonne d'échangeur d'anions AG1X4 (AcO⁻, 200-400 mesh, 2 x 16 cm). Après rinçage à l'eau bouillie, les **ACPT-I** (10 mg), **ACPT-II** (30 mg) et (±)**-ACPT-III** (120 mg) sont respectivement élués par une solution d'AcOH 0,5 M, 0,8 M et 1 M. Rendement global: 64,1 % par rapport au cétodiester.

### Analyse par CPV sous forme d'esters N-trifluoroacétyl-O,O'-diisopropyliques (M. Maurs, C. Ducrocq, A. Righini-Tapie et R. Azerad, J. Chromatogr., 25, 1985, 444-449):

Sur colonne capillaire OV-1701(Flexibond ®, Pierce) (15 m x 0.2 mm, 0.25 mm épaisseur de film) 190 °C: tR 13,5 min **[(±)-ACPT-III],** 16,4 min **(ACPT-II),** 19,2 min **(ACPT-I).**
Sur colonne capillaire Ultra-2 ® (Hewlett-Packard) (25 m x 0.2 mm; 0.33 mm épaisseur de film, 2 min à 140°C, puis gradient de 140 à 300°C (10°C/min), puis 5 min à 300°C: tR 13,7 min **((±)-ACPT-III),** 13,9 min **(ACPT-I),** 14,2 min **(ACPT-II).**
Sur colonne capillaire Chirasil-Val ® (50 m x 0.32 mm, 0.2 mm épaisseur de film) 190 °C: tR 15,0 min [(3S,4S)-**ACPT-III]** et 15,1 min **[(3R,4R)-ACPT-III],** 18,3 min **[(1S, 3R, 4S) - (ACPT-II)**], 22,4 min **[(1S, 3R, 4S) - (ACPT-I)].** 150°C: tR 84.7 min **[(3S,4S)-ACPT-III]** et 86,1 min **[(3R,4R)-ACPT-III].**
**(1S, 3R, 4S) ACPT-I.** GC / MS (EI) *m/z* (% du pic de base): 352 (4), 338(7), 296(30), 268(7), 250(26), 222 (100), 204(16), 177(27).
¹H RMN (D₂0, sel NH₄⁺): 3,20 (m, 2H, H-3, H-4), 2,63 (m, 2H, H-2, H-5), 2,24 (m, 2H, H-2, H-5).
¹³C RMN: 183,8 et 179,0 (CO), 70,0 (C-1), 50,8 (C-3, C-4), 42,1 (C-2, C-5).

| | | | |
|---|---|---|---|
| Anal.: calc pour C₈H₁₁NO₆, 2 H₂O, % | C 37,95; | H 5,97; | N 5,53 |
| trouvé, % | C 38,61; | H 5,46; | N 5,59. |

**(1R, 3R, 4S) ACPT-II.** GC / MS (EI) m/z (% du pic de base): 380(1), 352(12), 338(10), 310(3), 296(21), 268(5), 250(30), 222(100), 204(14), 177(22).
¹H RMN (D₂O, sel NH₄⁺): 3,33 (m, 2H, H-3, H-4), 2,52 (m, 2H, H-2, H-5), 2,32 (m, 2H, H-2, H-5).
¹³C RMN: 183,6 et 180,4 (CO), 69,2 (C-1), 53,7 (C-3, C-4), 42,3 (C-2, C-5)

| | | | |
|---|---|---|---|
| Anal: calc pour C₈H₁₁NO₆, 2 H₂O, % | C 37,95; | H 5,97; | N 5,53 |
| trouvé, % | C 38,53; | H 5,39; | N 5,57. |

**(±)-ACPT-III.** GC / MS (EI) *m/z* (% du pic de base): 440 (0.01), 380 (2), 352 (15), 338(8), 310 (14), 296 (12), 268 (65), 250 (20), 222 (100), 204 (18), 177 (28).
¹H RMN (D₂0, sel ⁺NH₄): 3,19 (m, 2H, H-3, H-4), 2,59, 2,42 et 2,10 (3m, 4H, H-2, H-5).
¹³C RMN: 185,9, 183,9 et 179,2 (CO), 69,9 (C-1), 53,2 (C-3, C-4), 43,3 et 42,8 (C-2, C-5)

| | | | |
|---|---|---|---|
| Anal: calc pour C₈H₁₁NO₆, 2 H₂O, % | C 37,95; | H 5,97; | N 5,53 |
| trouvé, % | C 38,47; | H 5,31; | N 5,54. |

### Exemple 2:

Au *cis* -cétodiester 5 (0,4 g, 2 mmol) dissous dans MeOH (4 ml) est traité par (NH₄)₂CO₃ (0,96 g, 5 eq.) et KCN (0,154 g, 1,2 eq.) comme décrit dans l'exemple 1. Le résidu obtenu après évaporation à sec est dissous dans une suspension de baryte (3,0 g dans 40 ml d'eau) dans une bouteille à vis et chauffé à 120 °C pendant 2 jours. La solution est refroidie, acidifiée avec une solution diluée d'H₂SO₄, filtrée sur célite, diluée avec 200 ml d'eau et déposée sur une colonne d'échangeur de cations Dowex 50X4 (H⁺, 20-50 mesh, 3 x 17 cm). La colonne est rinçée à l'eau (300ml) et le mélange des ACPT élué avec une solution d'ammoniaque 0,5 M. Les fractions positives à la ninhydrine sont évaporées (0,480 g) et le mélange redissous dans de l'eau bouillie (250 ml) est déposé à pH basique sur une colonne d'échangeur d'anions AG1X4 (AcO⁻, 200-400 mesh, 3 x 19 cm). Après rinçage à l'eau bouillie, les **ACPT-I** (17,2 mg), **ACPT-II** (32 mg) et (±)**-ACPT-III** (324 mg) sont élués par des solutions d'acide acétique comme décrit dans l'exemple 1. Rendement global: 75,1%.

### Exemple 3:

Au *cis*-cétodiester **5** (0,40 g, 2 mmol) dissous dans MeOH (4 ml) sont ajoutés (NH₄)₂CO₃ (0,96 g, 5 eq.) et KCN (0,154 g, 1,2 eq.) dissous dans l'eau (3 ml). Le mélange est chauffé à 55-60 °C avec réfrigérant pendant 2,5 h puis acidifié à pH 3-4 avec une solution d'HCl dilué. Après environ 15 min d'agitation à température ambiante, la solution est concentrée, diluée avec une solution aqueuse de NaCl (20 ml) et extraite à l'AcOEt (50 ml x 2). La phase organique est séchée sur Na₂SO₄, évaporée et séchée sous vide (0.407.g, 1.5 mmol, rendement brut 75.4 %). Au mélange d'hydantoines dissous dans CH₃CN (20 ml) on ajoute du di-t-butyldicarbonate (1.15 ml, 3 eq.) et de la DMAP (15 mg, 0,08 eq.) [Kubik, S.; Meissner, R. S.; Rebek Jr., J. *Tetrahedron Lett.* **1994,** 35, 6635-6638]. Après 1,5 h le solvant est évaporé, le résidu repris dans CH₂Cl₂, rapidement filtré sur une petite colonne de silice (2 x 13 cm) et élué avec CH₂Cl₂ (60 ml), puis CH₂Cl₂ / AcOEt (4:1) (150 ml). Le diBoc-ester détecté sur couche mince au TDM (VonArx, E.; Feyel, M.; Brugger, M. J. *J. Chromatogr.* **1976,** *120,* 224-228) est évaporé (0,533 g, 1,13 mmol) puis dissous dans CH₃CN (3 ml) et LiOH 1N (12 ml). La solution trouble est agitée pendant 5 h à température ambiante, refroidie à 0 °C, neutralisée avec HCl 1N (12 ml) et évaporée à sec. Le résidu est traité avec une solution d'HCl gazeux (~1N) dans l'acide acétique (0,5 h) puis évaporé à sec. Le mélange des **ACPT-I, II** et **III** est dissous dans l'eau (1 L) ajusté à pH 4 avec NaHCO₃ si nécessaire, purifié sur une colonne de Dowex 50X4 (H⁺, 20-50 mesh, 3 x 17 cm) (0,177 g) puis séparé sur AG1X4 (AcO⁻, 200-400 mesh) comme décrit précédemment, pour donner **ACPT-I** (0,035 g), **ACPT-II** (0,116 g) et **(±)-ACPT-III** (0,029 g). Rendement global: 36,1 %.

### Exemple 4:

Au *cis*-cétodiester **5** (0,4 g, 2 mmol) dissous dans l'eau (5 ml) sont ajoutés NH₄Cl (1,08 g, 10 eq.) et KCN (0,13 g, 1 eq.). Après 3 jours d'agitation à température ambiante, on ajoute (NH₄)₂CO₃ (0,211 g, 2,2 mmol, 1,1 éq.) et l'agitation est poursuivie pendant 30 h. La solution est ensuite acidifiée à pH 4 avec HCl 1N et évaporée à sec. Le résidu est transféré dans une bouteille à vis, mis en suspension dans une solution d'HCl / MeOH (8 ml) et chauffé 1,5 h à 60°C. Les sels insolubles sont filtrés, rincés avec MeOH et le filtrat évaporé et séché sous vide sur KOH (0,742 g). Le mélange brut d'esters d'hydantoines est dissous dans AcOEt (20 ml), lavé avec une solution saturée de NaCl à laquelle on ajoute NaHCO₃ pour maintenir un pH neutre. La phase aqueuse de lavage est réextraite avec AcOEt (10 ml). Les phases organiques réunies sont séchées sur Na₂SO₄ et évaporées. Au mélange d'esters méthyliques d'hydantoine dissous dans CH₃CN (25 ml) on ajoute du di-t-butyldicarbonate (1.5 ml, 3 eq.) et de la DMAP (21 mg, 0,08 eq.). La solution est agitée une nuit et rapidement purifiée par flash chromatographie (SiO₂, 230-400 mesh, 2 x 10 cm, éluant CH₂Cl₂, puis CH₂Cl₂/AcOEt, 4:1), conduisant à un rendement total de 53,5% (0,503 g, 1,07 mmol). Le mélange d'hydantoines totalement protégées est traité par LiOH, HCl/AcOH, purifié et séparé comme décrit précédemment: **ACPT-I** (0,062 g), **ACPT-II** (0,096 g), **(±)-ACPT-III** (0,035 g). Rendement global: 38,8% par rapport au cétodiester.

### Exemple 5:

Le *trans* -cétodiester (±)-**8** (0,2 g, 1 mmol) est traité comme décrit dans l'exemple 1 et conduit à l'**ACPT-I** (0,015 g), l'**ACPT-II** (0,02 g) et au **(±)-ACPT-III** (0,15 g). Rendement global: 74,2% par rapport au cétodiester.

### Exemple 6:

Le *trans* -cétodiester-(--)-(3R,4R)-**8** (0,134 g, 0,67 mmol, ee > 98%) est traité par (NH₄)₂CO₃ / KCN, comme décrit dans l'exemple 3 pendant 5 h, puis reestérifié comme décrit dans l'exemple 4 pour donner une huile incolore (0,149 g, 0,552 mmol, rendement 82%) qui est alors traitée par Boc₂O / DMAP puis LiOH et HCl/AcOH et purifiée comme décrit dans l'exemple 3. On obtient après la colonne d'échangeur de cations un mélange (0,091g, 54,1%) contenant essentiellement le **(-)-(3R,4R)-ACPT-III** (97,5%) qui est purifié par chromatographie sur échangeur d'anions (0,083 g, 0,333 mmol, ee 99,2%), et de faibles quantités d'**ACPT-I** (0,6%) et d'**ACPT-II** (1,9%).
**(--) (3R,4R)-ACPT-III :** [α]_{D}²² = -32,3 (c 0,73, H₂O).

### Exemple 7:

Le *trans* -cétodiester-(+)-(3S,4S)-**8** (0,172 g, 0,858 mmol, ee > 99,8%) est traité comme décrit dans l'exemple 6. On obtient après la colonne d'échangeur de cations un mélange (0,109 g, 50,6%) contenant essentiellement le **(+)-(3S,4S)-ACPT-III** (98,2%) qui est purifié par chromatographie sur échangeur d'anions (0,105 g, 0,451 mmol, ee 98,3%) et de faibles quantités d'**ACPT-I** (<0,4%) et d'**ACPT-II** (1,8%).
**(+)-(3S,4S)-ACPT-III:** [α]_{D} ²² = +26,4 (c 0,64, H₂O).

### Exemple 8 : Etude de l'activité biologique.

On a testé l'activité biologique des ACPT-I, -II et -III sur des cellules HEK 293 transfectées et exprimant mGluR1a, mGluR2 ou mGluR4a, choisis comme prototypes des récepteurs métabotropiques des groupes I, II ou III.

On a mesuré à cet effet l'activation de la phospholipase C (accumulation d'inositol phosphates).

### 1) Matériel et méthodes

### - Culture et transfection de cellules HEK 293

Les cellules HEK 293 sont cultivées dans un milieu Eagle modifié de Dulbecco (DMEM, Gibco BRL) supplémenté par 10 % de sérum de veau foetal et transfecté par électroporation comme précédemment décrit par Gomeza et al, Molecular Pharmacology, 1996, 50:923-930 (1996).

L'électroporation est effectuée dans un volume total de 300 *µ*l avec 10 *µ*g d'ADN support, de l'ADN plasmidique contenant mGluRla (0.3 *µ*g), mGluR2 (2 *µ*g) ou mGluR4a (5 *µ*g) et 10⁷ cellules.

Pour permettre l'activation de la phospholipase C par mGluR2 et mGluR4a, effet plus facile à mesurer que l'inhibition de la production de cAMP, ces récepteurs sont co-exprimés avec des protéines G chimères, Gqo5 et Gqi9, comme décrit par Gomeza. Les profils pharmacologiques de ces 2 récepteurs déterminés selon cette méthode, ont déjà été décrits comme identiques à ceux caractérisés en mesurant l'inhibition de la formation de cAMP.

### - Détermination de l'accumulation d'inositol phosphates (IP).

La détermination de l'accumulation d'IP dans les cellules transfectées est réalisée comme décrit par Gomeza après marquage des cellules pendant environ 14 heures avec du [³H]-myo-inositol (23,4 Ci/mol, NEN, France).

La stimulation est réalisée pendant 30 min. dans un milieu contenant 10 mM LiCl en présence des drogues testées. La formation de l'IP de base est déterminée après 30 min. d'incubation en présence de 10 mM LiCl et de pyruvate glutamate transaminase (1U/ml) dégradant le glutamate (Glu) et 2 mM de pyruvate pour éviter les effets possibles de Glu libéré par les cellules. Les résultats sont exprimés en quantité de IP produit par rapport à la radioactivité présente dans les membranes.

### - Culture et enregistrement des cellules granulaires du cervelet

Les cellules granulaires du cervelet sont cultivées à partir de bébés souris de 7 jours comme décrit par Van-Vliet, B.J., J. of Neurochemistry, 1989, .52, 1229-1239.

L'enregistrement est effectué en utilisant la technique dite "patch clamp" dans la configuration cellulaire entière et les molécules sont appliquées en utilisant une technique d'application rapide.

### 2/ Résultats

En premier lieu, des essais ont été réalisés pour vérifier si les ACPT étaient capables d'activer ou d'exercer un effet antagoniste vis-à-vis des récepteurs ionotropiques. Ces essais ont montré qu'aucun des énantiomères d'ACPT n'induit de courant lorsqu'on les met en contact avec les cellules granulaires du cervelet à une concentration de 1 mM.

Dans les mêmes lots, on observe au contraire des courants induits par le NMDA (100 *µ*M) ou le kainate (100 *µ*M, concentration qui permet une activation à la fois des récepteurs AMPA et kainate), comme prévu compte tenu de l'activation des récepteurs NMDA et non NMDA. Les effets antagonistes de l'ACPT-I, de l'ACPT-II et de l'ACPT-III (1mM) ont été également examinés sur des réponses induites par le NMDA ou le kainate (100 *µ*M). Aucune inhibition significative n'a été observée avec l'ACPT-II et l'ACPT-III. En revanche, on a pu noter une faible inhibition en présence d'ACPT-I (1 mM), de l'ordre de 10 %, des réponses induites par le NMDA ou le kainate.

L'effet des différents ACPT testés a été ensuite analysé sur des récepteurs mGluRs représentatifs des groupes I, II et III, à savoir mGluR1a, mGluR2 et mGluR4a.

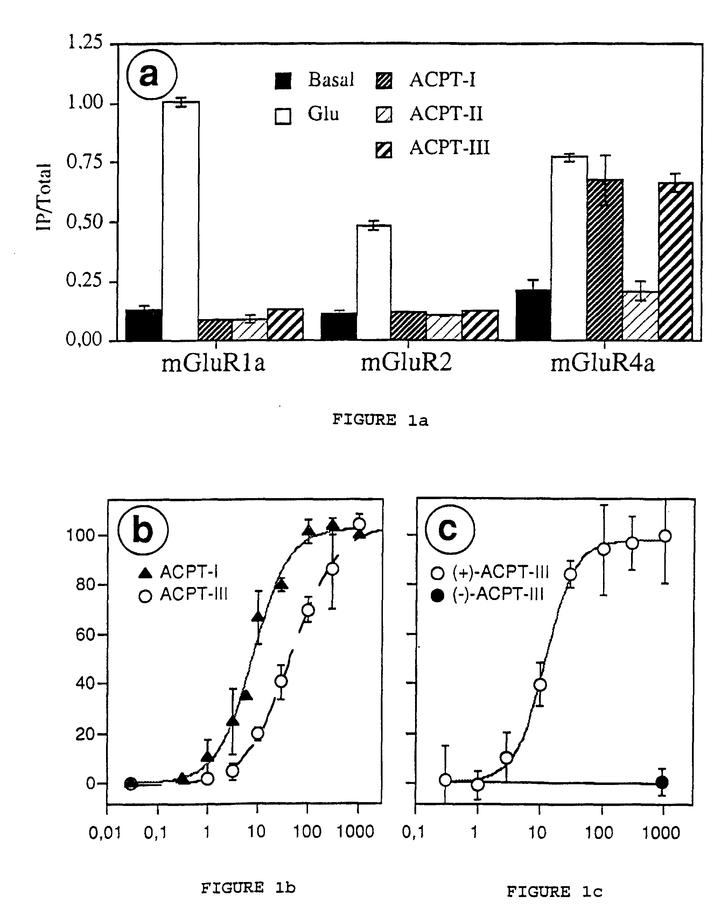

Les résultats obtenus sont illustrés par les Figures 1a à 1c et résumés dans le Tableau 2.

La figure 1a indique l'accumulation d'inositol phosphates (IP) induite par ces molécules (3mM), comparée à celle induite avec du (Glu) à une concentration de 1 mM. Sur cette figure, ■ représente les valeurs (IP) de base, □ celles mesurées en présence de Glu, 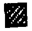 en présence d'ACPT-I, 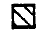 , en présence d'ACPT-II et 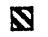 en présence d'ACPT-III.

Les figures 1b et 1c donnent la réponse de production des IP, exprimée en % de l'effet maximal, en fonction de la concentration de drogue utilisée en *µ*M.

La figure 1b indique l'effet agoniste de l'ACPT-I (▲) et du (±) ACPT-III (o), à différentes concentrations, sur mGluR4a et la figure 1c l'effet agoniste pour les énantiomères de l'ACPT-III (o:(+)ACPT-III ; ● : (-) ACPT-III, à différentes concentrations, sur mGluR4a.

Comme le montre la figure la, aucune des molécules d'ACPT n'active mGluRla ou mGluR2.

En revanche, l'ACPT-I et l'ACPT-III activent mGluR4a de manière dose-dépendante (figures la et 1b) avec des valeurs de CE₅₀ (concentration effective donnant 50 % de l'effet maximal), respectivement, de 7,2 ± 2,3 et de 40 ± 8 *µ*M (n=3).

On remarque que seul le (+) ACPT-III possède des propriétés agonistes vis-à-vis de mGluR4a avec une valeur de CE₅₀ de 8,8 ± 3,2 *µ*M (n=2) (Fig. 1c).

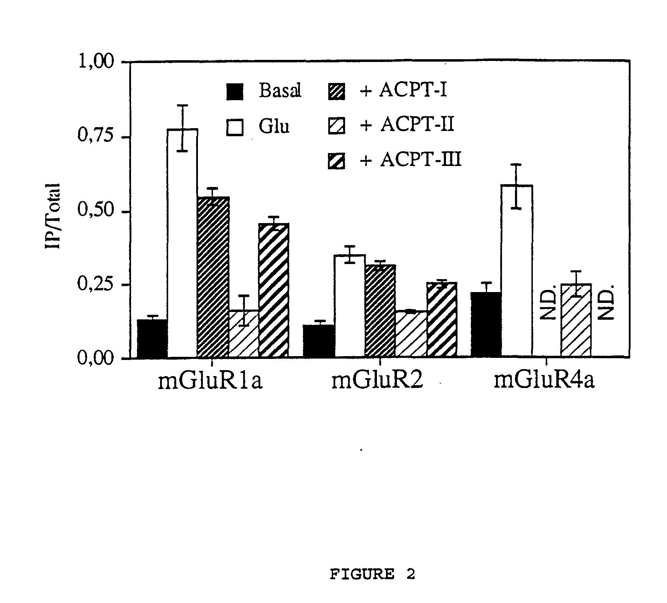

Sur la figure 2, on a représenté l'inhibition de la formation des IP induite par le Glu, sous l'effet des ACPT (les symboles sont les mêmes que dans la figure 1a).

On constate que les ACPT-I, -II, et -III inhibent la formation des IP induite par le Glu dans les cellules exprimant mGluR1a. Tous ces composés induisent un déplacement vers la droite de la courbe dose-réponse du Glu comme attendu pour des antagonistes compétitifs.

On constate que les composés ACPT-II et -III inhibent aussi les réponses induites par le Glu dans les cellules exprimant mGluR2. Ce résultat n'est pas obtenu avec l'ACPT-I. L'ACPT-II apparaît plus actif que l'ACPT-III.

On remarquera de plus que le composé ACPT-II exerce également un effet antagoniste vis-à-vis de l'effet du Glu sur mGluR4a et apparaît donc comme un antagoniste général des récepteurs glutamatergiques métabotropiques.

L'ensemble des résultats ci-dessus est résumé dans le tableau 2 suivant où est indiqué l'effet agoniste (CE₅₀, *µ*M) ou antagoniste (K_{B}, *µ*M) des ACPT sur les récepteurs mGluRla, mGluR2 et mGluR4a.

| | mGluR1a | | mGluR2 | | mGluR4a | |
|---|---|---|---|---|---|---|
| | CE₅₀ | K_{B} | CE₅₀ | K_{B} | CE₅₀ | K_{B} |
| **ACPT-I** | - | >1000 | - | - | 7.2 ± 2.3 | - |
| **ACPT-II** | - | 115 ± 2 | - | 88 ± 21 | - | 77 ± 9 |
| (±)-**ACPT-III** | - | >300 | - | >300 | 40 ± 8 | - |
| (+)-(3S,4S)-**ACPT-III** | - | (1000)^{*a*} | - | (150)^{*a*} | 8.8 ± 3.2 | - |
| (-)-(3R,4R)-**ACPT-III** | - | (1000)^{*a*} | - | (300)^{*a*} | - | 220 |
| "-" : pas d'activité à la plus forte concentration testée (3 mM). ^{*a*} Valeurs d'CI₅₀ (Glu= 20 µM pour mGluR1a et mGluR2, 30 µM pour mGluR4a) | | | | | | |

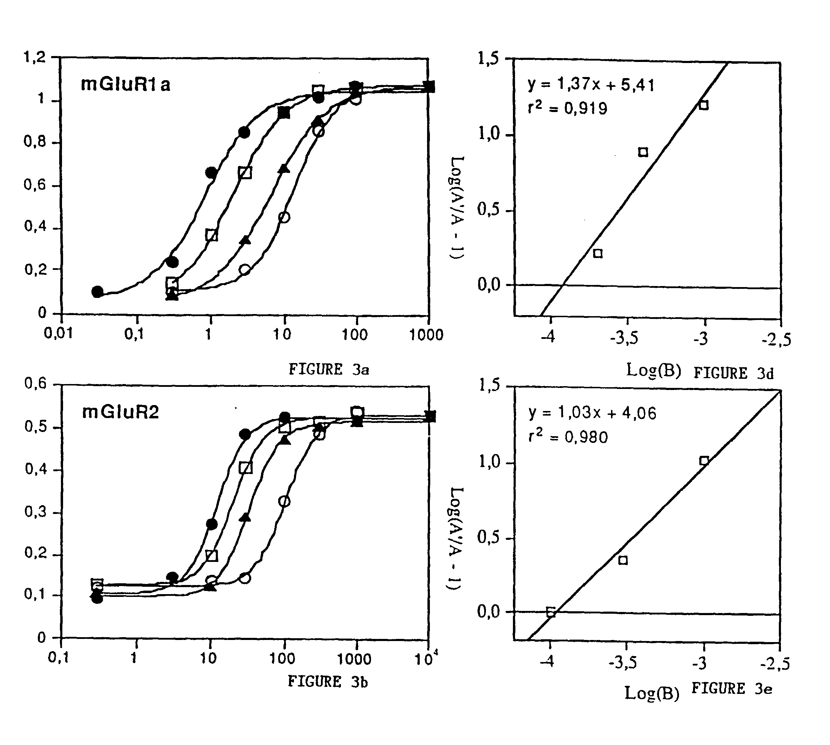

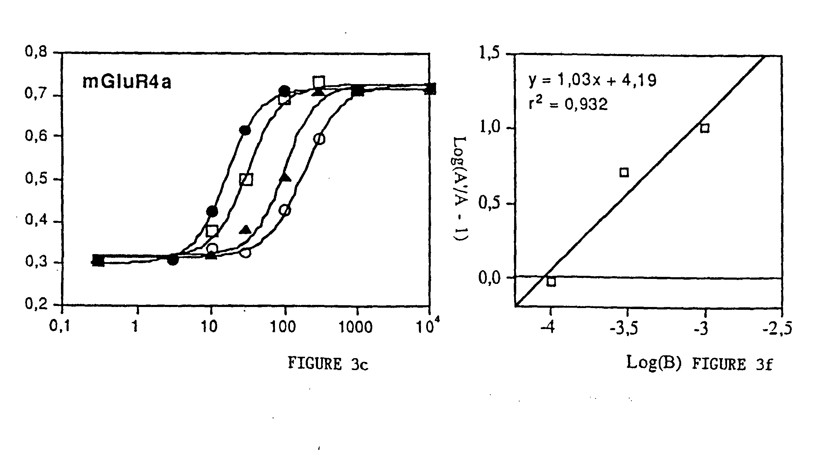

Les figures 3a à 3c et 3d à 3f illustrent l'effet antagoniste, et la détermination de la constante d'inhibition de l'ACPT-II sur les récepteurs mGluR1a (figures 3a et 3d), mGluR2 (figures 3b et 3e) et mGluR4a (figures 3c et 3f). Les figures 3a à 3c donnent la production d'IP (par rapport au total de la radioactivité, en valeurs brutes), en fonction de la concentration de Glu en *µ*M (échelle logarithmique), utilisé seul (témoin) ou en présence de concentration fixe d'ACPT-II. Les symboles ont les significations suivantes : (●), témoin ; (□), + 100 *µ*M (200 *µ*M pour mGluR1a) ; (▲), + 300 *µ*M (500 *µ*M pour mGluR1a) ; o, + 1000 *µ*M.

Les graphes des figures 3d à 3f montrent la représentation graphique de Shild obtenue à partir des courbes dose-réponses des figures 3a à 3c et donnent les valeurs de Log(A'/A-1) en fonction de Log (B), où A' est la valeur de CE₅₀ du Glu mesurée en présence de la concentration B d'ACPT-II et A, la valeur de CE₅₀ du Glu mesurée en l'absence d'ACPT-II, c'est-à-dire d'antagoniste.

On constate que le composé ACPT-II exerce un effet similaire sur mGluR1a, mGluR2 et mGluR4a. Dans chaque cas, la pente est proche de l'unité comme prévu pour un antagoniste compétitif. Les valeurs respectives KB déterminées grâce à la représentation de Shild sont : 115 ± 2, 88 ± 21, 77 ± 9 *µ*M (n = 3) respectivement pour mGluR1a, mGluR2 et mGluR4a.

On a également étudié l'effet du (+) ACPT-III et du (-) ACPT-III sur ces récepteurs, compte tenu de l'activité de l'ACPT-III, formé des deux énantiomères, sur mGluR1a et mGluR2.

L'effet antagoniste éventuel du composé (-)ACPT-III sur mGluR4a a été également étudié, étant donné que seul l'énantiomère (+) est un agoniste de ce récepteur comme indiqué ci-dessus.

Les résultats obtenus sont rapportés sur les figures 3a à 3c. Ces figures concernent respectivement les résultats obtenus sur mGluR1a, mGluR2 et mGluR4a. Les symboles sont les suivants : (●) effet antagoniste du composé (+) ACPT-III et (o) du composé (-) ACPT-III sur mGluRs. Les concentrations en ACPT-III sont exprimées en unités *µ*M.

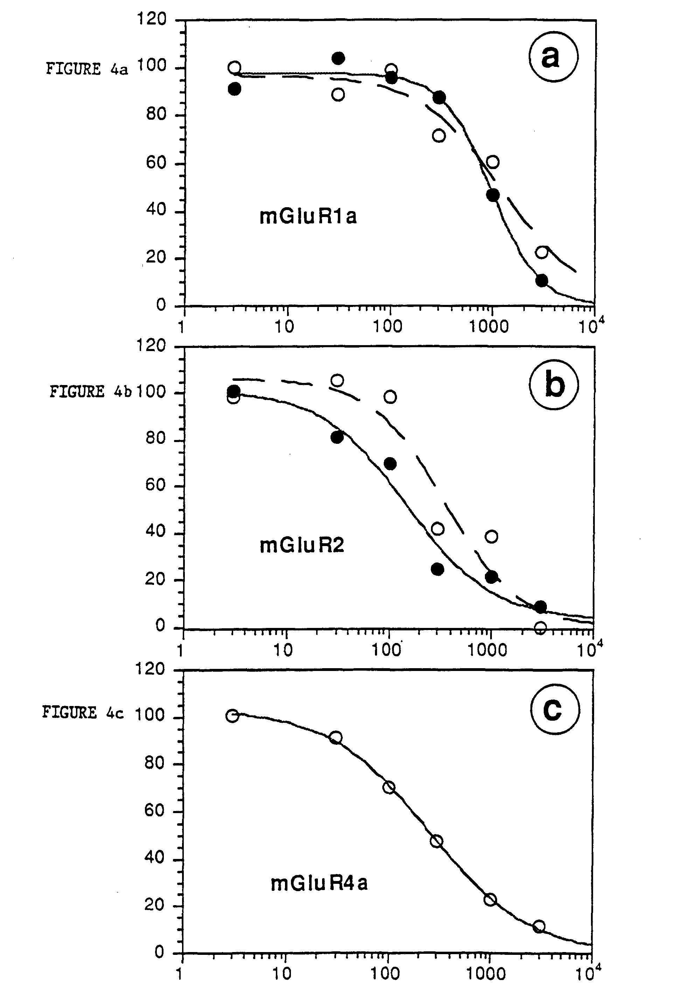

On constate que les ACPT-III (+) et (-) inhibent les réponses Glu, avec une faible puissance dans les cellules exprimant mGluRla ou mGluR2. Sur mGluR4a, le composé (-) ACPT-III exerce un effet antagoniste vis-à-vis de celui de Glu (figure 4c) et l'analyse des courbes Shild indique une inhibition compétitive (pente = 0,877) avec une valeur de K_{B} de 220 *µ*M.

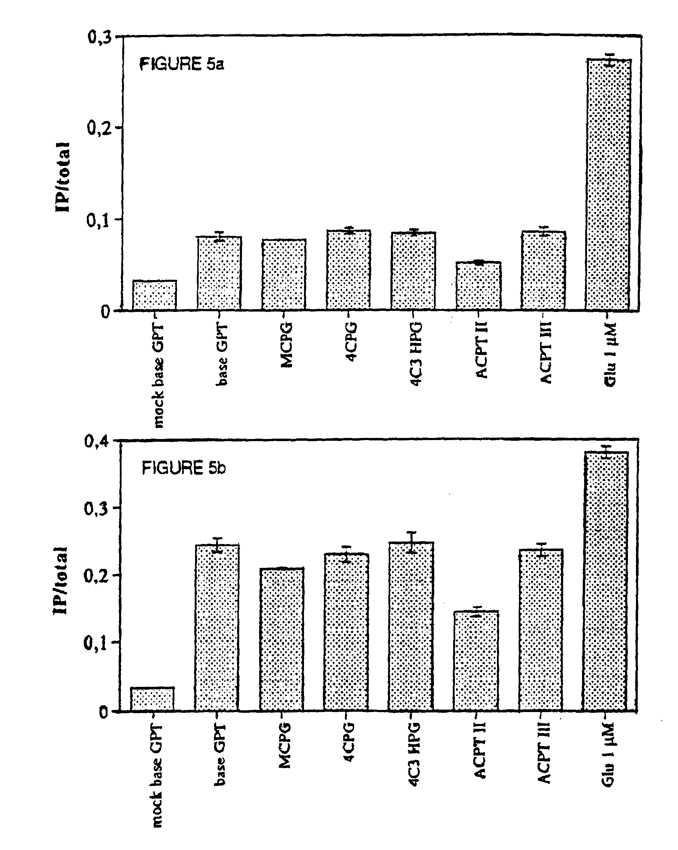

Comme déjà décrit avec de nombreux autres récepteurs couplés aux protéines G, il est possible de détecter une activité de base du récepteur mGluR1a (Prézeau et al., Molecular Pharmacology, 1996, 49:422-429). En effet, même en totale absence d'agoniste, une activité accrue de la phospholipase C est détectable dans les cellules exprimant mGluR1a. Cependant, contrairement à ce qui est observé avec d'autres récepteurs couplés aux protéines G, aucun des antagonistes compétitifs connus de mGluR1 n'est capable d'inhiber l'activité constitutive de ce récepteur. Aucune de ces molécules n'agit donc comme agoniste inverse. Par contre, l'application d'ACPT-II (mais pas celle d'ACPT-I ou d'ACPT-III) à une concentration de 1mM inhibe l'activité basale de mGluRla mesurée tant dans des conditions contrôles, ou lorsque le récepteur est co-exprimé avec la sous-unité alpha de la protéine Gq. Ces résultats sont rapportés sur les figures 5a et 5b. Sur la figure 5a, on voit que seul l'ACPT-II inhibe de manière significative l'activité basale de la phospholipase C mesurée sur des cellules HEK 293 exprimant le récepteur mGluR1a. La figure 5b montre les résultats obtenus sur des cellules exprimant mGluR1a et sur-exprimant la sous-unité alpha de la protéine Gq. Ces observations montrent que l'ACPT-II est le seul antagoniste de mGluR1a connu doué d'une activité agoniste inverse sur ce récepteur.

## Revendications

1. Composés possédant notamment des propriétés agonistes, antagonistes ou agonistes inverses vis-à-vis de récepteurs glutamatergiques, **caractérisés en ce qu'**ils répondent à la formule (I) 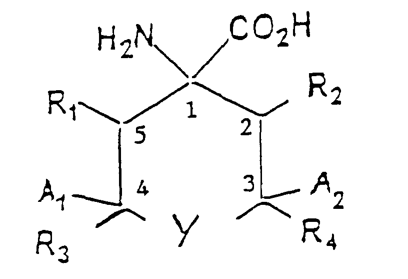 dans laquelle
- R₁ à R₄, identiques ou différents les uns des autres, représentent un atome d'hydrogène, un radical alkyle ou un radical aryle, ces radicaux étant le cas échéant eux-mêmes substitués, R₁ et R₂ ensemble pouvant en outre représenter un pont -(CH₂)ₘ, où m est un entier de 1 à 5,
- A₁ et A₂, représentent tous deux l'un des radicaux -COOH, -SO₃H ou -PO₃H₂, ou leurs dérivés tels qu'esters ou amides,
- Y représente une chaîne hydrocarbonée - (CH₂)ₙ -, n étant nul ou un entier de 1 à 5, ou un hétéroatome O, N ou S, ou une combinsaison linéaire de n groupes hydrocarbonés et au moins un hétéroatome O, N ou S.

2. Composés selon la revendication 1, **caractérisés en ce qu'**ils répondent à la formule I dans laquelle les carbones portant R₁ et R₂ ne sont pas reliés entre eux en formant un pont et Y n'existe pas.

3. Composés selon la revendication 1, **caractérisés en ce qu'**ils répondent à la formule I dans laquelle les carbones portant R₁ et R₂ ne sont pas reliés par un pont et Y représente un groupe -(CH₂)ₙ- où n est un entier de 1 à 5 et comporte le cas échéant au moins un hétéroatome O, N ou S.

4. Composés selon l'une quelconque des revendications précédentes, **caractérisés en ce que** A₁ et A₂ représentent tous deux un groupe carboxyle, ou ses dérivés tels qu'esters ou amides.

5. Composés selon l'une quelconque des revendications 1, 2 ou 4, **caractérisés en ce que** R₁ à R₄ représentent un atome d'hydrogène.

6. Composés, selon l'une quelconque des revendications précédentes, **caractérisés en ce qu'**il s'agit de molécules achirales ou chirales sous forme de différents diastéréoisomères, éventuellement comme racémique, le cas échéant sous forme de l'un des énantiomères.

7. Composés selon la revendication 5, **caractérisés en ce qu'**ils correspondent aux acides 1-amino-cyclopentane 1, 3, 4 tricarboxyliques ACPT-I, ACPT-II, ACPT-IIIa et ACPT-IIIb répondant respectivement aux formules (II), (III), (IVa) et (IVb) suivantes.

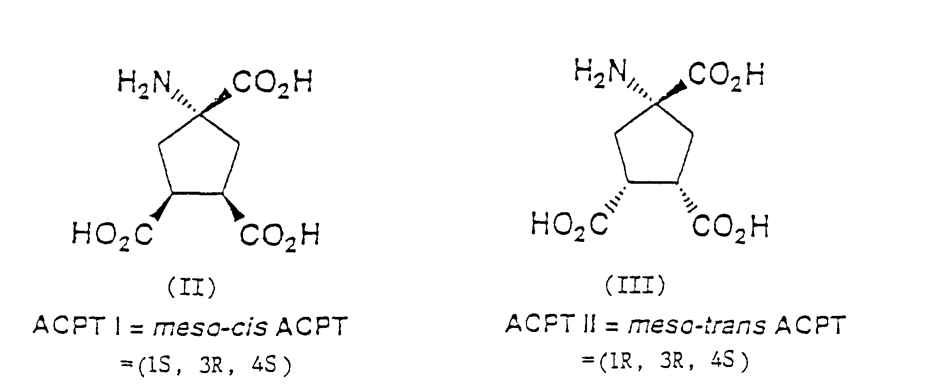

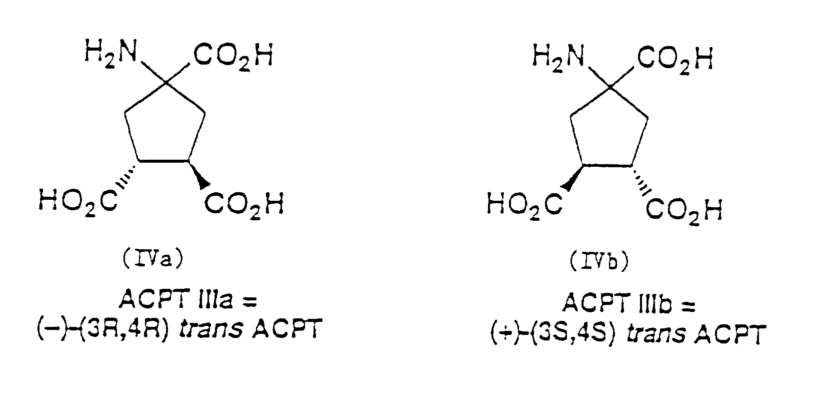

8. Procédé de synthèse de composés selon l'une quelconque des revendications 1 à 7, **caractérisé en ce qu'**il comprend :
- en opérant selon la technique de Bucherer-Bergs, la réaction d'une cétone de formule (V) 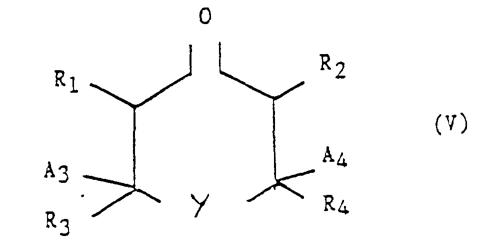 dans laquelle R₁ à R₄ et Y sont tels que définis dans la formule I et A₃ et A₄ représentent un groupe -COOR₅, PO₃(R₅)₂, PO₃HR₅, ou SO₃R₅, où R₅ est un atome d'hydrogène ou un radical alkyle,
avec un cyanure et un carbonate basique d'ammonium
- en opérant selon la technique de Strecker, la réaction de la cétone (V) avec un sel d'ammonium et un cyanure, puis l'addition de carbonate d'ammonium au mélange réactionnel, ces réactions étant réalisées dans des conditions conduisant à une hydantoïne de formule (VI) 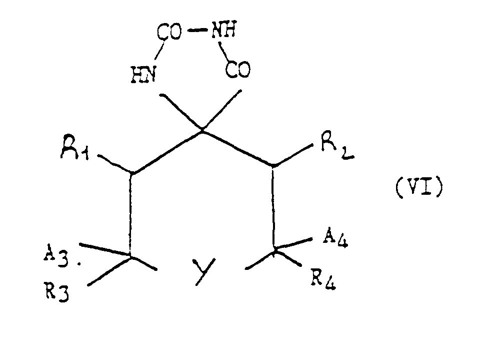
- suivi de l'hydrolyse de l'hydantoïne ainsi obtenue, ou d'une hydantoine dérivée dans laquelle les groupes -NH sont bloqués par des groupes protecteurs, cette hydrolyse conduisant à l'obtention d'un mélange renfermant divers diastéréoisomères,
- à la séparation des différents composés, et au dédoublement des racémiques si souhaité.

9. Composés selon l'une quelconque des revendications 1 à 7, pour utilisation comme agents agonistes, antagonistes ou agonistes inverses de récepteurs glutamatergiques métabotropiques.

10. Compositions pharmaceutiques, **caractérisées en ce qu'**elles renferment une quantité efficace d'au moins un composé selon l'une quelconque des revendications 1 à 7, en association avec un véhicule pharmaceutiquement acceptable.

## Claims

1. Compounds possessing in particular agonist, antagonist or reverse agonist properties vis-à-vis glutamatergic receptors, **characterized in that** they correspond to formula (I) 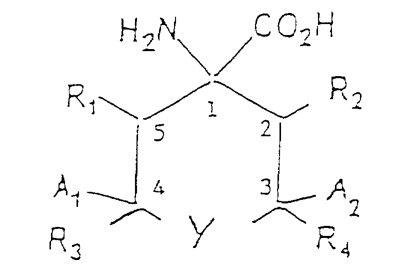 in which
- R₁ to R₄, identical to or different from each other, represent a hydrogen atom, an alkyl radical or an aryl radical, these radicals themselves being substituted where appropriate, R₁ and R₂ together being able to further represent a -(CH₂)ₘ bridge, where m is an integer from 1 to 5,
- A₁ and A₂ both represent one of the -COOH, -SO₃H or-PO₃H₂ radicals or their derivatives such as esters or amides,
- Y represents a hydrocarbon chain -(CH₂)ₙ-, n being nil or an integer from 1 to 5, or a heteroatom O, N or S, or a linear combination of n hydrocarbon groups and at least one heteroatom O, N or S.

2. Compounds according to claim 1, **characterized in that** they correspond to formula I in which the carbons carrying R₁ and R₂ are not linked together in forming a bridge and Y does not exist.

3. Compounds according to claim 1, **characterized in that** they correspond to formula I in which the carbons carrying R₁ and R₂ are not linked by a bridge and Y represents a - (CH₂)ₙ- group where n is an integer from 1 to 5 and where appropriate comprises at least one heteroatom O, N or S.

4. Compounds according to any one of the previous claims, **characterized in that** A₁ and A₂ both represent a carboxyl group, or its derivatives such as esters or amides.

5. Compounds according to any one of claims 1, 2 or 4, **characterized in that** R₁ to R₄ represent a hydrogen atom.

6. Compounds according to any one of the previous claims, **characterized in that** they are achiral or chiral molecules in the form of different diastereoisomers, optionally as racemic, where appropriate in the form of one of the enantiomers.

7. Compounds according to claim 5, **characterized in that** they correspond to 1-amino-cyclopenlane 1, 3, 4 tricarboxylic acids, ACPT-I, ACPT-II, ACPT-IIIa and ACPT-IIIb respectively corresponding to the following formulae (II), (III), (IVa) and (IVb).

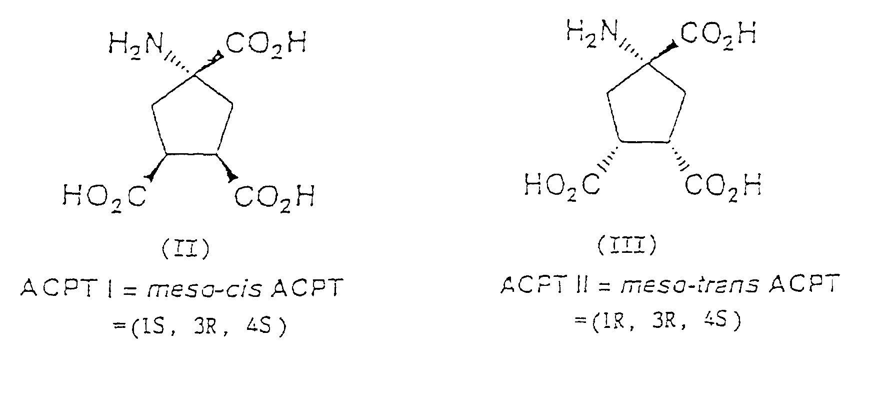

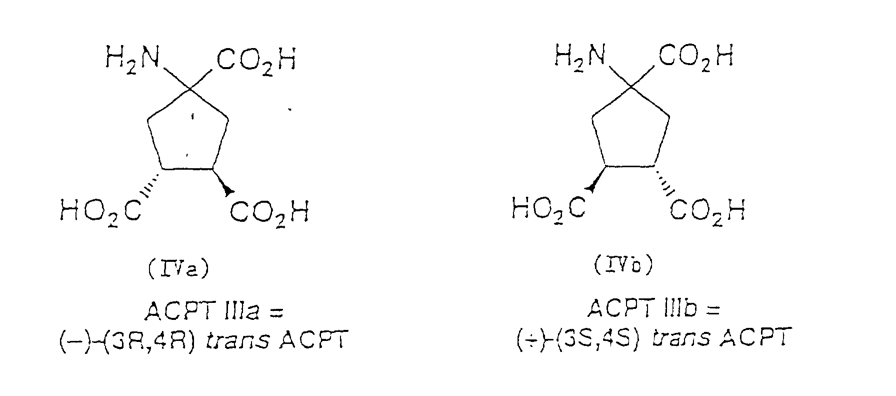

8. Process for synthesizing compounds according to any one of claims 1 to 7, **characterized in that** it comprises:
- operating according to Bucherer-Bergs' technique, the reaction of a ketone of formula (V) 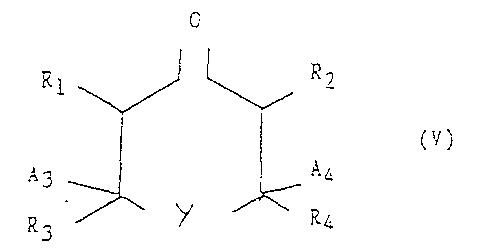 in which R₁ to R₄ and n are as defined in formula I and A₃ and A₄ represent a -COOR₅, PO₃(R₅)₂, PO₃HR₅, or SO₃R₅ group, where R₅ is a hydrogen atom or an alkyl radical,
with a cyanide and a basic ammonium carbonate,
- operating according to Strecker's technique, the reaction of the ketone (V) with an ammonium salt and a cyanide, then the addition of ammonium carbonate to the reaction mixture, these reactions being carried out under conditions leading to a hydantoin of formula (VI) 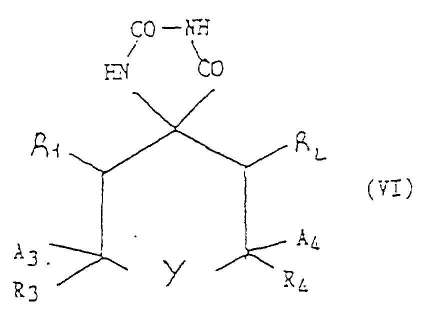
- followed by hydrolysis of the hydantoin thus obtained, or of a hydantoin derivative in which the -NH groups are blocked by protective groups, this hydrolysis leading to obtaining a mixture containing various diastereoisomers,
- to the separation of the different compounds, and the resolution of the racemics if desired.

9. Compounds according to any one of claims 1 to 7, for use as agonist, antagonist or reverse agonist agents of metabotropic glutamatergic receptors.

10. Pharmaceutical compositions **characterized in that** they contain an effective quantity of at least one compound according to any one of claims 1 to 7, in combination with a pharmaceutically acceptable vehicle.

## Patentansprüche

1. Verbindungen, die insbesondere agonistische, antagonistische oder inverse agonistische Eigenschaften gegenüber Glutamat-Rezeptoren besitzen, **dadurch gekennzeichnet, dass** sie der Formel (I) 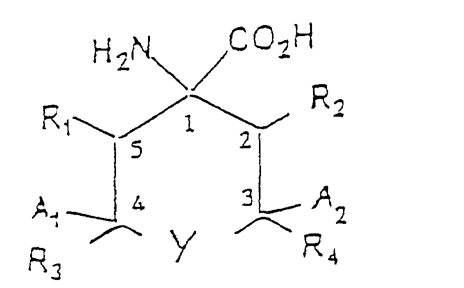 entsprechen, in welcher
- R₁ bis R₄, die gleich oder voneinander verschieden sind, ein Wasserstoffatom und einen Alkyl- oder Arylrest bedeuten, wobei diese Reste gegebenenfalls selbst substituiert sind und R₁ und R₂ zusammen außerdem eine Brücke -(CH₂)ₘ-, worin m ganzzahlig von 1 bis 5 ist, bedeuten können,
- A₁ und A₂ beide einen der Reste -COOH, -SO₃H oder -PO₃H₂ bzw. deren Derivate wie Ester oder Amide bedeuten und
- Y eine Kohlenwasserstoffkette -(CH₂)ₙ-, worin n gleich Null oder eine ganze Zahl von 1 bis 5 ist, ein Heteroatom O, N bzw. S oder eine geradkettige Kombination von n Kohlenwasserstoffgruppen mit mindestens einem Heteroatom O, N bzw. S bedeutet.

2. Verbindungen nach Anspruch 1, **dadurch gekennzeichnet, dass** sie der Formel I entsprechen, in welcher die R₁ und R₂ tragenden Kohlenstoffatome nicht unter Brückenbildung miteinander verbunden sind und Y nicht vorhanden ist.

3. Verbindungen nach Anspruch 1, **dadurch gekennzeichnet, dass** sie der Formel I entsprechen, in welcher die R₁ und R₂ tragenden Kohlenstoffatome nicht durch eine Brücke miteinander verbunden sind und Y eine -(CH₂)ₙ-Gruppe, worin n ganzzahlig von 1 bis 5 ist, bedeutet und gegebenenfalls mindestens ein Heteroatom O, N bzw. S umfasst.

4. Verbindungen nach einem der vorhergehenden Ansprüche, **dadurch gekennzeichnet, dass** A₁ und A₂ beide eine Carboxylgruppe oder deren Derivate wie Ester bzw. Amide bedeuten.

5. Verbindungen nach einem der Ansprüche 1, 2 oder 4, **dadurch gekennzeichnet, dass** R₁ bis R₄ ein Wasserstoffatom bedeuten.

6. Verbindungen nach einem der vorhergehenden Ansprüche, **dadurch gekennzeichnet, dass** es sich um achirale oder chirale Moleküle in Form verschiedener Diastereoisomerer, gegebenenfalls als Racemat oder in Form eines der Enantiomeren handelt.

7. Verbindungen nach Anspruch 5, **dadurch gekennzeichnet, dass** sie den 1-Aminocyclopentan-1,3,4-tricarbonsäuren ACPT-I, ACPT-II, ACPT-IIIa und ACPT-IIIb entsprechen, welche die folgenden Formeln (II), (III), (IVa) und (IVb) besitzen:

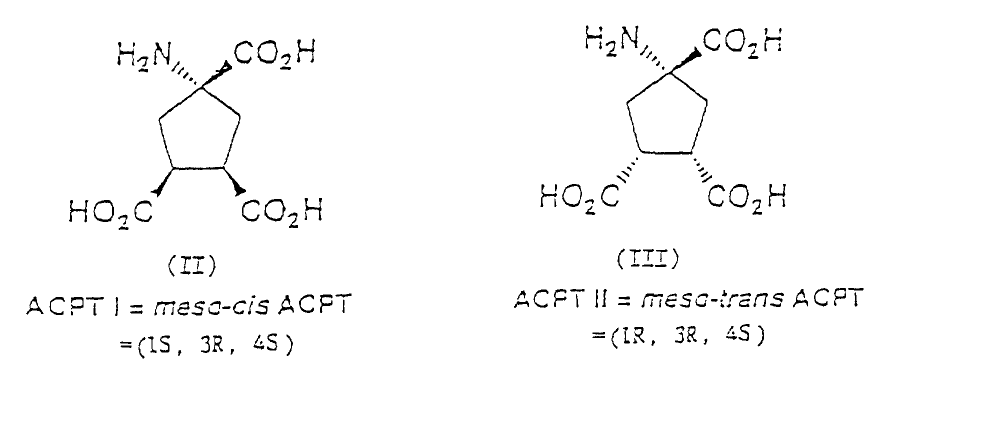

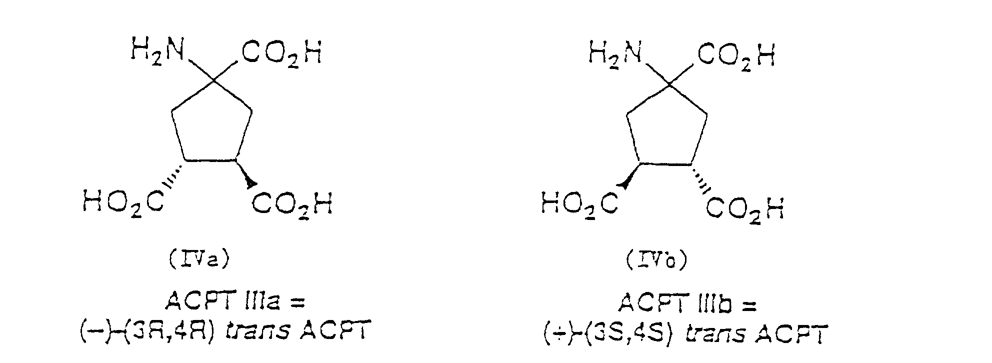

8. Verfahren zur Herstellung von Verbindungen nach einem der Ansprüche 1 bis 7, **dadurch gekennzeichnet, dass** es
- gemäß der Bucherer-Bergs-Reaktion die Umsetzung eines Ketons mit der Formel (V) 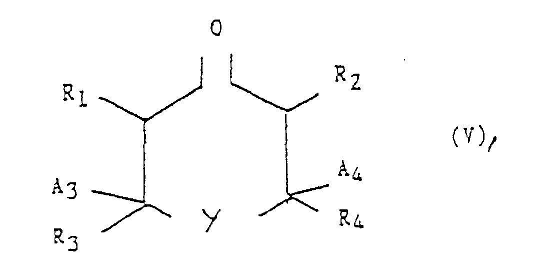 in welcher R₁ bis R₄ und Y wie in Formel I definiert sind und A₃ und A₄ eine -COOR₅-, PO₃(R₅)₂-, PO₃HR₅- bzw. SO₃R₅-Gruppe bedeuten, worin R₅ ein Wasserstoffatom oder einen Alkylrest bedeutet, mit einem Cyanid und einem basischen Ammoniumcarbonat oder
- gemäß der Strecker-Synthese die Umsetzung des Ketons (V) mit einem Ammoniumsalz und einem Cyanid und danach die Zugabe von Ammoniumcarbonat zum Reaktionsgemisch, wobei diese Umsetzungen unter Bedingungen durchgeführt werden, die zu einem Hydantoin mit der Formel (VI) führen, 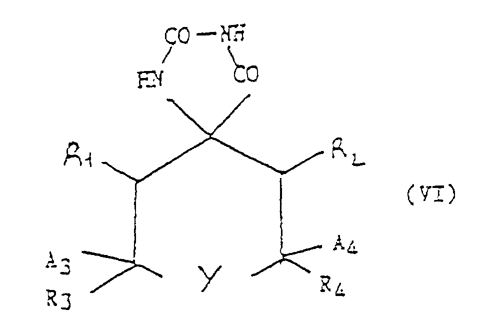
- die anschließende Hydrolyse des so erhaltenen Hydantoins bzw. eines Hydantoinderivats, in welchem die -NH-Gruppen durch Schutzgruppen blockiert sind, wobei die Hydrolyse zum Erhalt eines Gemischs führt, das verschiedene Diastereoisomeren umfasst, und
- die Abtrennung der verschiedenen Verbindungen und, falls gewünscht, Auftrennung des Racemats
umfasst.

9. Verbindungen nach einem der Ansprüche 1 bis 7 zur Verwendung als agonistische, antagonistische oder inverse agonistische Mittel an metabotropen Glutamat-Rezeptoren.

10. Pharmazeutische Zusammensetzungen, **dadurch gekennzeichnet, dass** sie eine wirksame Menge mindestens einer Verbindung nach einem der Ansprüche 1 bis 7 zusammen mit einem pharmazeutisch verträglichen Träger umfassen.
